# EUROPEAN PATENT SPECIFICATION

(11) **EP 4 452 059 B1**
(45) Date of publication and mention of the grant of the patent: **16.09.2026**
(21) Application number: 22854356.7
(22) Date of filing: 20.12.2022
(51) Int. Cl.: A61B 5/053, A61B 5/0245, A61B 5/00, G16H 50/30, G16H 15/00, G16H 50/20, G16H 40/63, A61B 5/08, A61B 5/085, G16H 20/40, G16H 20/30

(54) **CENTRAL APNEA DETECTION**
ZENTRALER APNOE-NACHWEIS
DÉTECTION D'APNÉE CENTRALE

(30) Priority: 21.12.2021 US 202163265817 P
(43) Date of publication of application: 30.10.2024
(73) Proprietor: Medical Informatics Corp., Houston, Texas 77004 (US)
(72) Inventor: WAUGH, Jamie L. S., Houston, Texas 77004 (US); PATEL, Raajen J., Houston, Texas 77004 (US); SAHEBA, Anuj Amit, Houston, Texas 77004 (US); RUSIN, Craig, Houston, Texas 77030 (US)
(74) Representative: Knöner, Gregor
(86) International application number: PCT/US2022/082072
(87) International publication number: WO 2023/122622

(56) References cited:
- EP-B1- 0 647 426
- US-A- 4 379 460
- US-A- 4 781 201
- US-A1- 2012 172 730

## Description

### TECHNICAL FIELD

The present invention relates to the field of detection of apnea, and in particular to the detection of central apnea.

### BACKGROUND ART

Apnea is the temporary cessation of breathing. Apnea can occur at any stage of development and is particularly common in premature infants, where it may be called apnea of prematurity (AOP). AOP is quite different from adult sleep apnea. While AOP is a developmental disorder, the reasons behind the propensity for apnea in immature infants are not entirely clear. Although the pathogenesis of AOP is poorly understood, the immature pulmonary reflexes and breathing responses to hypoxia and hypercapnia likely contribute to the occurrence or severity of AOP. It may also be exacerbated by coexisting factors or disease states.

AOP is an important and common clinical problem and is often the rate-limiting process in Neonatal Intensive Care Unit (NICU) discharge. Accurate detection of episodes of clinically important neonatal apnea using existing chest impedance monitoring is a clinical imperative.

Apneas are serious clinical events that need immediate medical attention. However, existing monitors for apnea, particularly for neonatal infants, are unsatisfactory-they miss many serious events. For this reason, they often fail to recognize apnea and therefore fail to provide a warning signal to NICU personnel alerting them to the fact that the infant is not breathing.

US 2012/0172730 A1 discloses a respiratory monitoring system and method for improved detection and response to apnea.

US 4,781,201 discloses an adaptive filtering device for suppressing a cardiovascular artifact from a respiratory signal derived from a patient's transthoracic impedance.

Thus, there is a need in the art for techniques that provide improved detection of apneas.

### SUMMARY

The invention is defined in the independent claims. The dependent claims describe embodiments of the invention.

### BRIEF DESCRIPTION OF DRAWINGS

The accompanying drawings, which are incorporated in and constitute a part of this specification, illustrate an implementation of apparatus and methods consistent with the present disclosure and, together with the detailed description, serve to explain advantages and principles consistent with the disclosure. In the drawings,
Figure 1 is a screenshot illustrating a graphical user interface for displaying an indication of an apnea event according to one embodiment.
Figure 2 is a screenshot illustrating a graphical user interface for displaying an indication of an apnea event according to one embodiment.
Figure 3 is a flowchart illustrating a technique for detecting an apnea event according to one embodiment.
Figure 4 is a block diagram illustrating a system for collecting, archiving, and processing arbitrary data in a healthcare environment according to one embodiment.
Figure 5 is a block diagram illustrating a computer server for use in the system of FIG. 4.
Figure 6 is a screenshot illustrating a graphical user interface for tracking apnea events according to one embodiment.

### DETAILED DESCRIPTION

In the following description, for purposes of explanation, numerous specific details are set forth in order to provide a thorough understanding of the invention. References to numbers without subscripts are understood to reference all instances of subscripts corresponding to the referenced number. Reference in the specification to "one embodiment" or to "an embodiment" means that a particular feature, structure, or characteristic described in connection with the embodiments is included in at least one embodiment of the disclosure, and multiple references to "one embodiment" or "an embodiment" should not be understood as necessarily all referring to the same embodiment.

Although some of the following description is written in terms that relate to software or firmware, embodiments can implement the features and functionality described herein in software, firmware, or hardware as desired, including any combination of software, firmware, and hardware. References to daemons, drivers, engines, modules, or routines should not be considered as suggesting a limitation of the embodiment to any type of implementation.

Apnea is a serious clinical event that needs medical attention within seconds and is very common in premature infants. Newborns tend to have an irregular breathing pattern and stop breathing for a few seconds at a time. Physicians differ in how they determine which events are of clinical importance. A common rule of thumb considers apnea to be a clinical event if (a) cessation of breathing lasts for more than 20 seconds or (b) cessation of breathing lasts for more than 10 seconds and is accompanied by either bradycardia (slowing of the heart) or oxygen desaturation. In neonates, bradycardia is typically defined as a heart rate less than 100 beats per minute and oxygen desaturation is typically defined as an SpO₂ less than 80%. Bradycardia and desaturation events can occur without a cessation of breathing. When these vital thresholds are passed within a defined period of the start or end of a neonate's significant cessation of breathing event, further deterioration has likely occurred.

Clinical apneas occur in more than half of babies whose birth weight is less than 1500 g, and in almost all infants whose birth weight is less than 1000 g. Apnea may be a cause or an effect of many other clinical problems, such as hypoxemia, hypoglycemia, neurological injury, or sepsis. Apnea is also a common manifestation of an immature neurological system in babies who were born very preterm but otherwise have no clinical pathology. Three types of apnea are common in premature infants: obstructive apnea, central apnea, and mixed apnea. Obstructive apnea is a blockage of the airway, typically accompanied by struggling or thrashing movements of the infant. Central apnea is the cessation of respiratory drive, and the infant makes no effort to breathe and usually remains very still. Mixed apneas typically begin with an obstructive event, and then change to central apnea. On the other hand, there is evidence that central apnea can also become obstructive apnea, so some sources suggest that the distinction between the two types should not be held too rigidly. In many cases, the apnea is combined with, or induces, bradycardia (a significant slowing of the heart rate). When accompanied by bradycardia, oxygen desaturation, or both, these apneas are serious clinical events that need immediate medical attention and investigation for associated pathology. Moreover, central apnea is taken to indicate immaturity of control of respiration, and discharge from NICUs is typically delayed until apneas have been absent for 3 to 8 days. These regulations exist so that infants can be safely discharged by reducing the likelihood that they will have another apnea episode once they are at home, without monitoring devices and clinicians.

Bedside monitors attempt to detect apnea based on continuous monitoring of chest impedance (CI) physiological data. Using electrodes that are also used to monitor the electrocardiogram (ECG), a small high-frequency (e.g., 52.6 kHz) voltage is applied to the chest, and the resulting high-frequency current is measured. The measured impedance Z is equal to the applied voltage V divided by the observed current. The measured impedance is typically in the range of 50 to 300 ohms and is related to the conductivity of muscle, skin, other tissues, and the contacts between electrodes and skin. When the infant is breathing, the impedance fluctuates with each breath. In a central apneic event, the chest impedance changes are reduced because of the cessation of breathing. However, the beating of the heart also causes fluctuations in impedance as every heartbeat pumps blood out of the thorax. Thus, even during an apneic event, the chest impedance fluctuates and can reach thresholds for detection as breaths, foiling the monitor's apnea alarm.

Detection of cessation of breathing in neonates is particularly difficult because a neonate's more malleable rib cage moves as its heart beats. Chest impedance leads may mistake the resulting change in rib cage diameter for respiration. Current apnea detectors do not address this problem so in these cases, clinicians must rely on alarms that indicate subsequent deterioration of the patient such as bradycardia.

As a result of these cardiac artifacts, some episodes of central neonatal apnea are missed altogether, and some of those episodes are severe. Some studies have found that conventional apnea monitors miss over 10% of even extreme apnea events because of cardiac artifacts in the chest impedance signal.

The techniques described below remove these cardiac artifacts from the chest impedance waveform used to measure respiration. Although described below in terms of real-time physiological data, the same techniques may be used on historical physiological data for retrospective analysis.

Due to the increased detection of apnea events, in certain embodiments, automated interaction may be utilized to stimulate the premature infant during an apnea event.

The disclosure below describes techniques for improving the detection of central apnea. More specifically, the standard CI signal used to monitor respiration rate is analyzed to filter out the contribution to the chest impedance signal that arises from a beating heart, and an indication of a breathing cessation event is indicated.

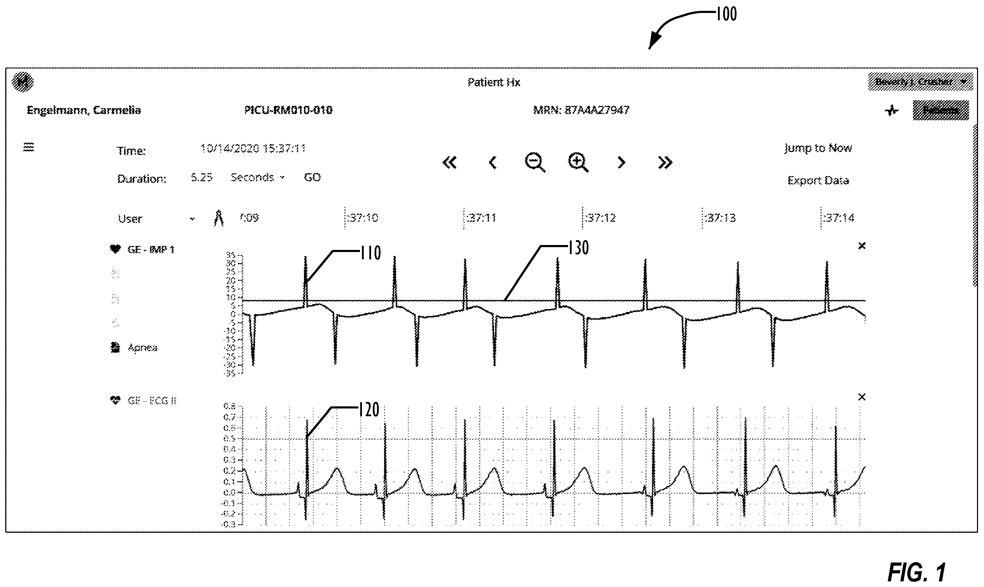

FIG. **1** is a screenshot illustrating a graphical user interface **100** according to one embodiment in which an indication of central apnea is displayed. Two waveform lanes are illustrated in the graphical user interface **100:** a CI waveform **110** and an ECG waveform **120.** The example waveform data in FIG. 1 is not intended to be medically accurate. In this example, the patient is experiencing an apnea condition, indicated by line **130,** even though the CI waveform **110** is not flat. The CI waveform is experiencing fluctuations as a result of the heartbeat illustrated in the ECG waveform **120.** We know this because both waveforms repeat cyclically at the same rate. In some embodiments, one or both of an audible alarm and a visual alarm indication may be generated in response to a determination that an apnea condition exists, in addition to the indication shown in line **130.** In other embodiments, an automatic response may be triggered to physically stimulate the infant, which may cause cessation of the apneic event.

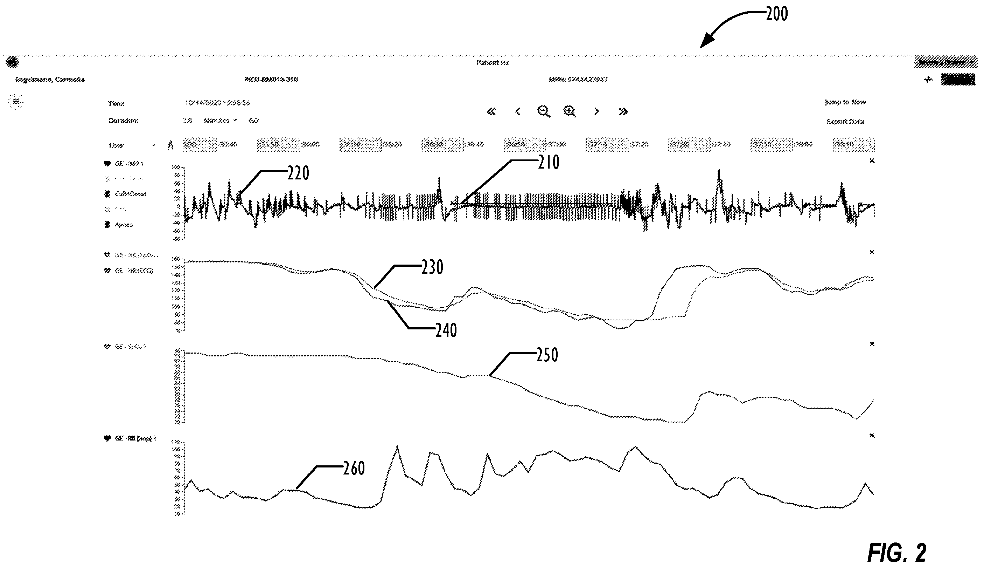

FIG. **2** is a screenshot illustrating a graphical user interface **200** according to one embodiment similar to the graphical user interface **100.** In this example, graph **220** is an example Cl waveform, graph **230** indicates a heart rate as determined from photoplethysmogram data, graph **240** indicates a heart rate as determined from ECG data, graph **250** indicates SpO₂ data determined from photoplethysmogram data, and graph **260** indicates a calculated respiration rate based solely on the Cl waveform. In this example, an apnea event is shown as indicated by horizontal line **210,** indicating that the patient ceased breathing and met bradycardia and oxygen desaturation criteria. In one implementation, apnea events are indicated as beginning only after a threshold time (e.g., 20 seconds) during which a cessation of breathing was identified and bradycardia and oxygen desaturation occurred. This reduces nuisance alarm indications for short apnea events that terminate without intervention.

While both FIG. 1 and FIG. 2 illustrate a real-time display of information showing the presence of an apnea event, some embodiments, in which real-time data is archived for research or clinical purposes may provide the ability to investigate historical patient data, analyze that data, and indicate the presence of apnea events in the historical data. Such historical investigation may be useful to detect prior apneas that were unnoticed and provide clinicians with better information to evaluate the health of the patient.

For clarity, the description below focuses on cessation of breathing events as being apnea events. However, different medical personnel may define apnea events as a cessation of breathing without other factors or a cessation of breathing combined with one or more other factors such as bradycardia or oxygen desaturation. The system may be configured for any combination of those additional factors as desired. For example, a patient may cease breathing for more than a threshold time, but maintain a normal heart rate and oxygen saturation, in which case some configurations of the system described below may not indicate an apnea event. Embodiments of the system described below may allow clinical personnel to select what combination of criteria are to be used for determining an apnea event.

Inputs to the central apnea detector may include a CI waveform, an ECG waveform, and heart rate and oxygen saturation (SpO₂) vitals. Each of the signals may undergo cleaning to eliminate data artifacts if desired.

The cardiac artifact component of the chest impedance can be identified based on its shared phase (progression of frequency) with any signal that captures heart movement, (e.g., an ECG signal), as described below. The first step is to model the progression of the cardiac phase. This can be done in multiple ways, using any signal that captures heart movement. For example, the cardiac phase could be calculated by taking the phase of the Hilbert transform applied to the photoplethysmogram waveform.

In one implementation, to determine the cardiac phase standard peak/trough detection is performed to find the R peaks in the ECG signal that occur each cardiac cycle. A cardiac phase signal *ϕ* is made to model the heart's frequency at any given time by setting the phase to 0 at each R peak time and having the phase increase linearly with time to 2π = 0 radians for samples in between each set of consecutive R peaks. Thus, the cardiac phase signal *ϕ* for a segment of data that contains N samples is an Nx1 vector $ϕ = \left[\begin{matrix}{ϕ}_{1} \\ {ϕ}_{2} \\ ⋮ \\ {ϕ}_{N}\end{matrix}\right]$

Components of the measured chest impedance signal *CIᵢₙᵢₜ* that correspond to the cardiac phase (i.e., the cardiac artifact) may then be extracted using a Fourier series approximation of *CIᵢₙᵢₜ* that is a function of the determined cardiac phase. Although the description below is written in terms of Fourier series, other frequency or time domain analysis and approximation techniques may be used.

The Fourier series approximation uses M harmonics stored as a 1xM vector $H = \left[1 2 ⋯ M\right]$

In some embodiments, M=3 sufficiently captures the frequency components contained in the cleaned Cl, but other numbers of harmonics may be used as desired.

This leads us to an NxM cardiac phase harmonic matrix *ϕH,* where each column represents the cardiac phase progression of a particular harmonic of the Fourier series representation of the cardiac artifact: $ϕH = \left[\begin{matrix}{ϕ}_{1} & 2 {ϕ}_{1} & ⋯ & {Mϕ}_{1} \\ {ϕ}_{2} & 2 {ϕ}_{2} & ⋯ & {Mϕ}_{2} \\ ⋮ & ⋮ & ⋱ & ⋮ \\ {ϕ}_{N} & 2 {ϕ}_{N} & ⋯ & {Mϕ}_{N}\end{matrix}\right]$

The frequency components F of the cardiac artifact Fourier series approximation can be calculated from the cardiac phase, resulting in an Nx2M matrix: $\begin{matrix}F = \left[cos\left(ϕH\right) sin\left(ϕH\right)\right] \\ = \left[cos\left(ϕ\right) cos\left(2ϕ\right) ⋯ cos\left(Mϕ\right) sin\left(ϕ\right) ⋯ sin\left(Mϕ\right)\right] \\ = \left[\begin{matrix}cos \left({ϕ}_{1}\right) & cos \left(2{ϕ}_{1}\right) & ⋯ & cos \left({Mϕ}_{1}\right) & sin \left({ϕ}_{1}\right) & ⋯ & sin \left({ϕ}_{1}\right) \\ cos \left({ϕ}_{2}\right) & cos \left(2{ϕ}_{2}\right) & ⋯ & cos \left({Mϕ}_{2}\right) & sin \left({ϕ}_{2}\right) & ⋯ & sin \left({Mϕ}_{2}\right) \\ ⋮ & ⋮ & ⋱ & ⋮ & ⋮ & ⋮ & ⋮ \\ cos \left({ϕ}_{N}\right) & cos \left(2{ϕ}_{N}\right) & ⋯ & cos \left({Mϕ}_{N}\right) & sin \left({ϕ}_{N}\right) & ⋯ & sin \left({Mϕ}_{N}\right)\end{matrix}\right]\end{matrix}$

We may then solve for the cardiac artifact Fourier series coefficients using the above cardiac artifact frequency components and the chest impedance signal. The Fourier series coefficients can be represented as a 2Mx1 vector: $\left[\begin{matrix}α \\ β\end{matrix}\right] = \left[\begin{matrix}{α}_{1} \\ {α}_{2} \\ ⋮ \\ {α}_{M} \\ {β}_{1} \\ {β}_{2} \\ ⋮ \\ {β}_{M}\end{matrix}\right]$

The cardiac artifact Fourier series coefficients are calculated as follows: $\left[\begin{matrix}α \\ β\end{matrix}\right] = {F}^{- 1} {CI}_{init}$

The cardiac artifact *CIₐᵣₜ* approximation may then be calculated as follows: $\begin{matrix}{CI}_{art} = F \left[\begin{matrix}α \\ β\end{matrix}\right] \\ = {\sum }_{m = 1}^{M} \left[{α}_{m} cos\left(mϕ\right)+{β}_{m} sin\left(mϕ\right)\right] \\ = \left[\begin{matrix}{\sum }_{m = 1}^{M} \left[{α}_{m} cos\left({mϕ}_{1}\right)+{β}_{m} sin\left({mϕ}_{1}\right)\right] \\ {\sum }_{m = 1}^{M} \left[{α}_{m} cos\left({mϕ}_{2}\right)+{β}_{m} sin\left({mϕ}_{2}\right)\right] \\ ⋮ \\ {\sum }_{m = 1}^{M} \left[{α}_{m} cos\left({mϕ}_{N}\right)+{β}_{m} sin\left({mϕ}_{N}\right)\right]\end{matrix}\right]\end{matrix}$

The cardiac artifact *CIₐᵣₜ* may then be removed from the measured chest impedance signal *CIᵢₙᵢₜ.* producing a filtered chest impedance signal *CI_{filt}* ${CI}_{filt} = {CI}_{init} - {CI}_{art}$

Once the cardiac artifact has been removed from the chest impedance, standard signal processing techniques of the filtered chest impedance and thresholding of the heart rate and oxygen saturation vitals can be used to identify cessation of breathing, bradycardia, and desaturation events. When there is little lung movement (small standard deviation) in the filtered chest impedance signal, the neonate is considered not breathing and a cessation of breathing event is identified.

An apnea event probability may be estimated using a Fermi function such as the following: ${P}_{fit} \left(σ\right) = \frac{1}{1 + {e}^{\left(12\left(σ-0.44\right)\right)}}$
where *σ* is the standard deviation of *CI_{filt}.* The parameters of the above Fermi function are illustrative and by way of example only. Other Fermi functions or types of functions may be used to calculate the probability of an apnea based on *CI_{filt}.* In some embodiments, when the apnea event probability exceeds a predetermined threshold value, the system is triggered and displays an alarm indication of an apnea event in a graphical user interface such as the ones illustrated in FIGs. 1 and 2. Furthermore, a set of alarm criteria may be developed based on the relative apnea event probability over a defined period. In addition, in embodiments where one or more of bradycardia and desaturation are considered apnea criteria, the result of measurement of heart rate and SpO₂ data may be combined with the probability calculated above to trigger the alarm indication.

When these vital thresholds are passed within a defined period of the start or end of a cessation of breathing event, a cessation of breathing with bradycardia and desaturation event is identified. The techniques described herein may be used for determining apneic events in children and adults, although the specific criteria such as heart rate and oxygen desaturation may be different in non-neonate groups.

The resulting improved system for detecting apnea events avoids missing apnea events that are masked by patient heartbeats in existing apnea detection systems.

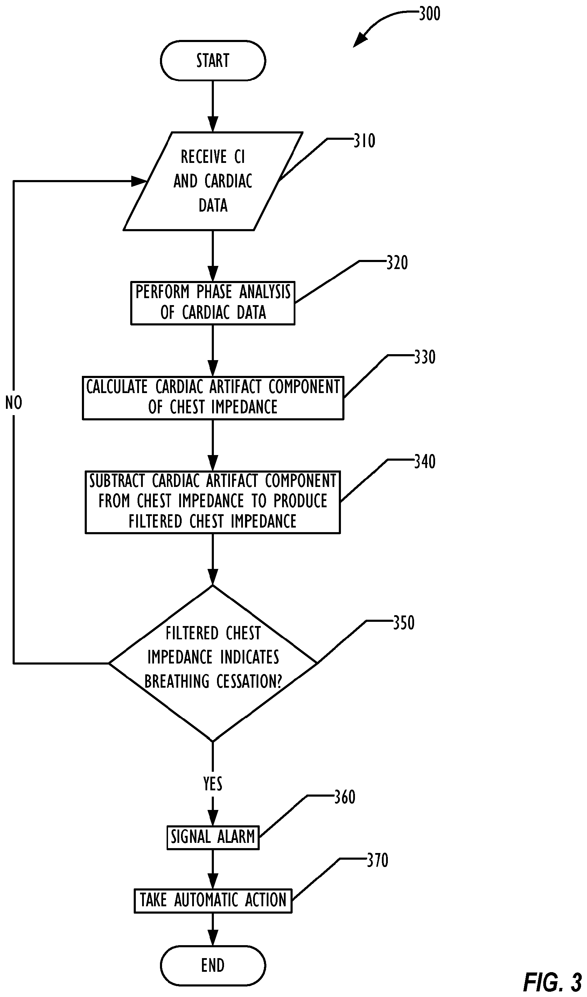

FIG. **3** is a flowchart illustrating a technique **300** for detecting and automatically acting on an apnea event according to one embodiment. In block **310,** the system receives real-time CI and cardiac data from sensors attached to a patient. A phase analysis of the cardiac data (e.g., ECG) is performed in block **320** as described above to create a phase signal *ϕ* and cardiac artifact phase harmonic matrix *ϕH.* In block **330,** using the algorithm described above, the cardiac artifact component of the chest impedance *CIₐᵣₜ* is calculated. Then in block **340,** the filtered chest impedance *CI_{filt}* is calculated by subtracting the cardiac artifact component *CIₐᵣₜ* from the measured chest impedance *CIᵢₙᵢₜ*. In block **350** if the filtered chest impedance *CI_{filt}* indicates breathing cessation an alarm may be triggered in the graphical user interface such as those illustrated in FIGs. **1-2****,** as an audible alarm, or in any other way known in the art for generating alarms. In some embodiments, a breathing cessation may be detected if the probability of an apnea event calculated as described above, reaches or exceeds a predefined threshold value or meets a set of criteria over a predefined duration. In some implementations, the overall periods in which the threshold is met are evaluated, rather than using a strict time threshold. For example, if there are two 12-second periods in which the threshold is met separated by one second, those two segments may be combined into one cessation of breathing event spanning 25 seconds.

If no breathing cessation is detected in block 350, the technique continues receiving CI and cardiac data in block **310.** If a cessation of breathing is detected in block **350,** an alarm is signaled in block **360.** In block **370,** where available, an automatic action may be taken to physically stimulate the patient responsive to the apnea event, such as creating a vibration, blowing air, or initiating another physical stimulus that may cause the patient to restart breathing. Where the system implementing technique **300** is configured to use one or more of bradycardia and desaturation as part of the threshold evaluation of an apnea event, technique **300** may include other actions not illustrated in FIG. 3 for detecting bradycardia and desaturation from heart rate and SpO₂ data received from patient monitors. Where no automatic physical stimulation of the patient is available, a clinical staff member may approach the patient to provide tactile stimulation manually, such as a gentle tap to the sole of the foot or rubbing the back. In some cases, where physical stimulation does not initiate spontaneous respiration, an apnea event may require additional interventions, such as ventilation with oxygen.

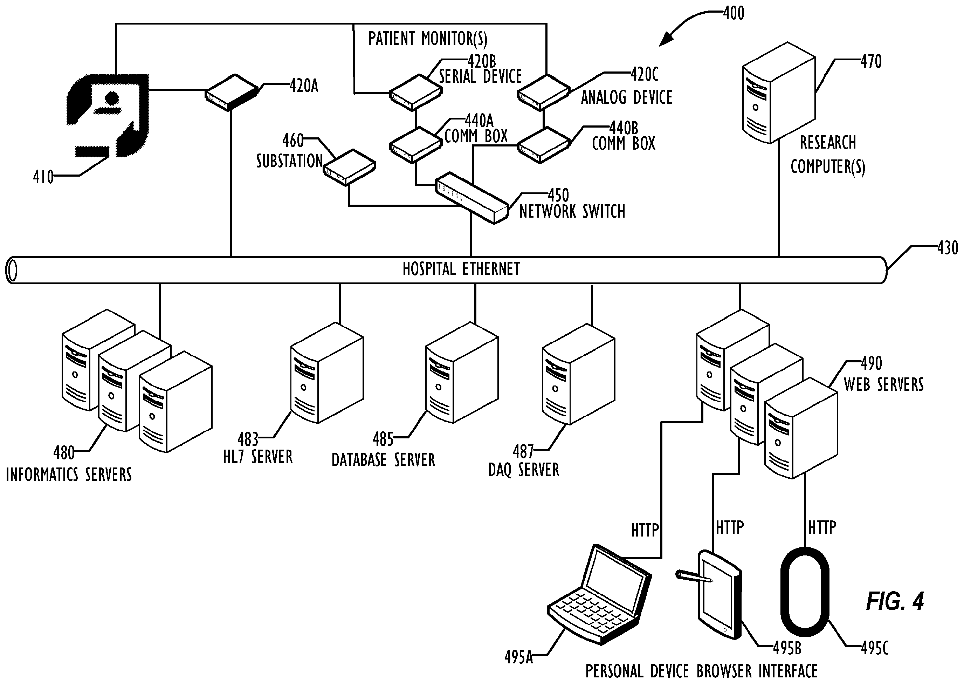

FIG. **4** is a block diagram illustrating a system **400** for collecting, archiving, and processing arbitrary data in a healthcare environment according to one embodiment. The system **400** is described in more detail in U.S. Pat. No. 10,892,045, "Distributed Grid-Computing Platform for Collecting, Archiving, and Processing Arbitrary Data in a Healthcare Environment".

As illustrated, there are five types of servers: the data acquisition (DAQ) server **487,** the informatics server(s) **480,** the database server **485,** the HL7 server **483,** and the web server (s) **490.** Any number of any of the types of servers may be deployed as desired. All of the servers **480-490** connect to each other and the bedside monitors via one or more hospital networks **430.** Although illustrated as a single hospital Ethernet network **430,** any number of interconnected networks may be used, using any desired networking protocols and techniques.

Also connected to the hospital network **430** are a number of bedside monitors for monitoring physiological data of a patient in bed **410.** These bedside monitors may include network-connected monitors **420A,** which can deliver digital physiological data to the hospital network **430,** serial devices **420B,** which produce digital data but are not directly connected to a network, and analog devices **420C,** which produce analog data and are not directly connected to a network. Communication boxes **440A** and **440B** allow connecting the serial devices **420B** and analog devices **420C,** respectively, to the hospital network **430,** typically through a network switch **450.** In addition, a substation **460** may also be connected to network **430** via the network switch **450** for performing data manipulation and time synchronization as described below. Any number of bedside monitors **420** may be used as determined advisable by physicians and other clinical staff for the patient in bed **410.**

Although FIG. **4** illustrates the bedside monitors and associated communication devices connected directly or indirectly to the hospital network **430,** remote bedside monitoring devices may be used as part of the system **400,** such as home monitoring devices, connected to the hospital network **430** indirectly through the Internet or other communication techniques.

Additionally, one or more research computers **470** may be connected, directly or indirectly, to the hospital network **430,** allowing researchers to access aggregated data collected from bedside monitors **420** for performing analytics and development.

The webservers **490** are configured for communicating with personal devices such as laptop **495A,** tablet **495B,** or smartphone **495C** via a web browser interface using HyperText Transport Protocol (HTTP).

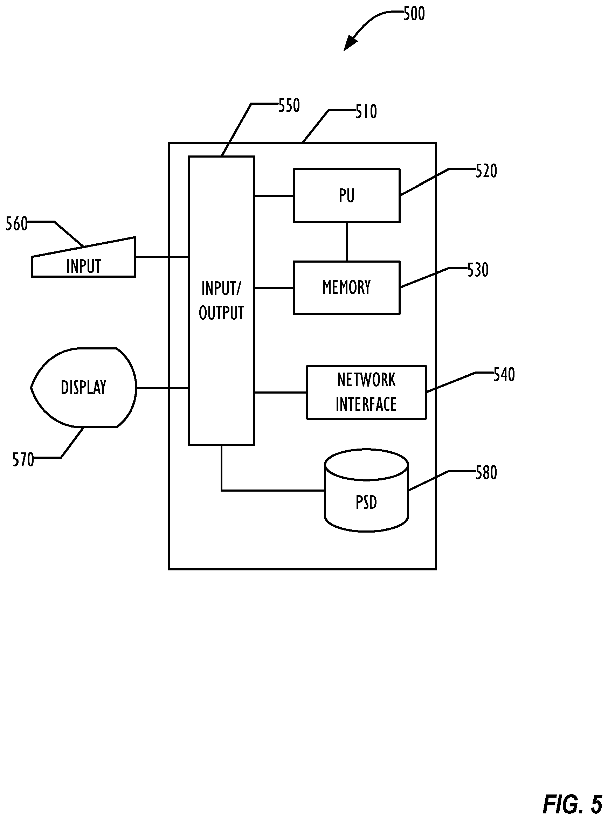

Referring now to FIG. **5****,** an example computer **500** for use as one of the servers **480-490** is illustrated in block diagram form. Example computer **500** comprises a system unit **510** which may be optionally connected to an input device or system **560** (e.g., keyboard, mouse, touch screen, etc.) and display **570.** A program storage device (PSD) **580** (sometimes referred to as a hard disc) is included with the system unit **510.** Also included with system unit **510** is a network interface **540** for communication via a network with other computing and corporate infrastructure devices (not shown). Network interface **540** may be included within system unit **510** or be external to system unit **510.** In either case, system unit **510** will be communicatively coupled to network interface **540.** Program storage device **580** represents any medium of non-volatile storage including, but not limited to, all forms of optical and magnetic, including solid-state, storage elements, including removable media, and may be included within system unit **510** or be external to system unit **510.** Program storage device **580** may be used for storage of software that when executed controls system unit **510,** data for use by the computer **500,** or both.

System unit **510** may be programmed to perform methods in accordance with this disclosure (an example of which is in FIG. **3****).** System unit **510** comprises a processor unit (PU) **520,** input-output (I/O) interface **550,** and memory **530.** Processor unit **520** may include any programmable controller device, such as microprocessors available from Intel Corp. and other manufacturers. Memory **530** may include one or more memory modules and comprise random access memory (RAM), read-only memory (ROM), programmable read-only memory (PROM), programmable read-write memory, and solid-state memory. One of ordinary skill in the art will also recognize that PU **520** may also include some internal memory including, for example, cache memory.

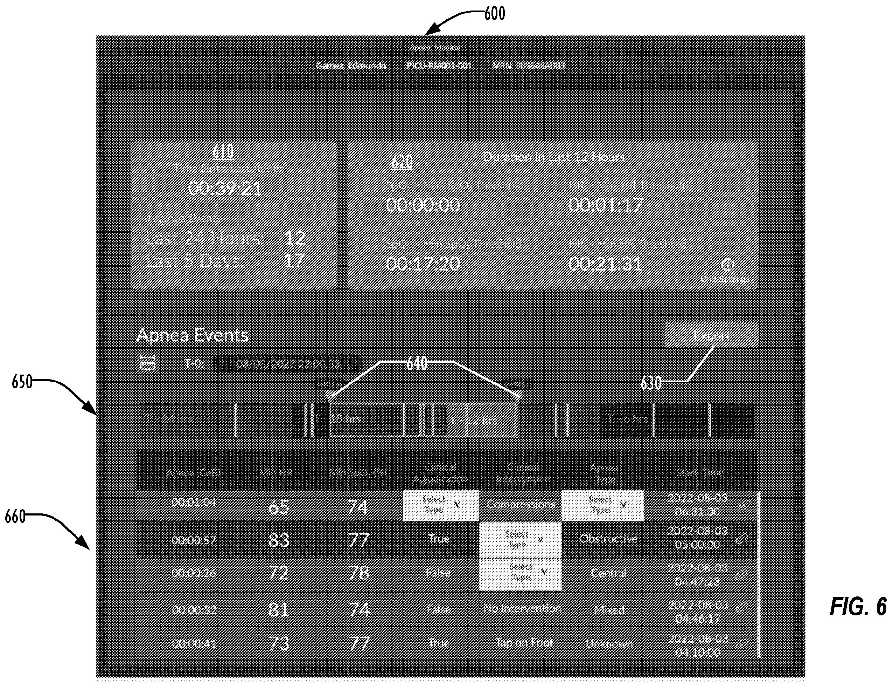

FIG. 6 is a screenshot illustrating an example graphical user interface 600 for monitoring historical information about apnea events for a patient according to one embodiment. Each apnea event is detected and recorded using the systems and techniques described above and may then be sent to the graphical user interface 600 for display. Aggregate information about the apnea events is also sent to the graphical user interface 600 for display. In this example graphical user interface 600, a first area 610 provides historical information about the number of recent apnea events, in this example a time since the last apnea event and the number of apnea events in the last 24 hours and five days. The display of "last 24 hours" and "last 5 days" is by way of example only, and other useful historical periods could be provided in addition to or instead of the ones illustrated in FIG. 6. A second area 620 provides historical information about the total length of apnea events in the last 12 hours. In this example, two types of oxygen saturation (SpO₂)-related records are displayed: SpO₂ > Max SpO₂ Threshold and SpO₂ < Min. SpO₂ Threshold. Similarly, two types of heart rate (HR)-related records are displayed: HR > Max HR Threshold and HR < Min. HR Threshold. Other types of historical information may be displayed in the second area 620 in addition to or instead of the information illustrated in FIG. 6.

Below the first area 610 and the second area 620 is an area providing historical information about individual apnea events. A timeline 650 in this example provides indicators of apnea events in the period 24 hours up to a selected "time zero." Other time ranges could be displayed if desired. A pair of calipers 640 may be placed on the timeline to select a particular window for detailed display in area 660 below the timeline 650. An Export button 630 allows exporting information about the selected time window to an electronic medical records (EMR) system.

In the detailed information area 660, details about each apnea event may be displayed. As illustrated in FIG. 6, the information displayed for each event includes the length of the cessation of breathing, a minimum HR occurring within a specified period relative to the event, a minimum oxygen saturation level occurring within a specified period relative to the event, whether a clinician adjudicated the event as detected by the system as a real apnea event, the type of clinical intervention that was performed in response to the apnea event, such as compressions, a tap on a foot, or no intervention, a designation of the type of apnea event, such as obstructive, central, mixed, or unknown, and a start time of the event. Other detailed information may be provided as desired in the user interface 600.

The areas and arrangement of areas in the example graphical user interface 600 of FIG. 6 are illustrative and by way of example only, and other areas and arrangements of areas may be provided as desired. Although some of the elements of the graphical user interface are illustrated as drop-down type user interaction elements, other types of user interaction elements may be used as desired. Color may be used to improve the user experience. For example, in some implementations, the indicator in timeline 650 of a particular apnea event may be displayed in a different color than the other indicators, while the corresponding row in the details area 660 is highlighted or indicated in a different color.

In clinical settings without the disclosed patient monitoring system, nurses or other clinical personnel manually record apnea events that occur on their shift in a flow sheet, either by handwriting data on a piece of paper or by typing data into an electronic form. In either event, the flow sheet requires clinical attention and is subject to human transcription error. The disclosed system allows automatically populating clinical flow sheets with apnea event information, which can significantly reduce both the manual labor of the nurse or other clinical personnel, as well as reducing the possibility of human error in recording the information. This may include the length of cessation of breathing, the start time, and SpO₂ and HR information. Other items, such as clinical adjudication, type of apnea, and the type of clinical intervention, might still be entered by the nurse, but would be entered in a standardized form by selecting from a list of possible entries. For example, clinical personnel report that detecting the end of an apnea event and thus calculating the length of the apnea event can be difficult. By automatically detecting both the beginning and end of the apnea event, the disclosed system may automatically calculate the length of the event, further reducing the load on clinical personnel and reducing manual data entry errors.

By collecting objective data on apnea events, instead of depending on detection by clinical personnel and improving on existing alarm systems, better tracking of apnea events may be provided. This improved tracking results in fewer false alarms but also in the detection of apnea events not currently detected in clinical settings. One result of such better data may be reduced clinical stays for infants, reducing costs of childcare both for the clinical facility and for the parents, as well as improving the quality of life of both the infant and the parents. Because patients often are not released until a certain amount of apnea-free time passes, reducing false alarms of apnea events can allow patients to be released earlier, for example.

While certain exemplary embodiments have been described in detail and shown in the accompanying drawings, it is to be understood that such embodiments are merely illustrative of and not devised without departing from the basic scope thereof, which is determined by the claims that follow.

## Claims

1. A patient monitoring system (400) for detecting apnea events in a patient, comprising:
bedside monitoring equipment (420A, 420B, 420C) associated with the patient that is configured to produce chest impedance physiological data and cardiac physiological data;
a computer system (500) programmed to:
receive the chest impedance physiological data and the cardiac physiological data (310);
model a cardiac phase using the cardiac physiological data (320);
calculate a frequency or time domain approximation of a cardiac artifact based on a representation of the modeled cardiac phase and the chest impedance physiological data (330);
remove the cardiac artifact from the chest impedance physiological data, producing a filtered chest impedance data (340);
calculate a probability of an apnea event based on the filtered chest impedance data (350); and
generate an alarm indication responsive to a set of criteria being met based on the probability of the apnea event over a period of time (360).

2. The patient monitoring system (400) of claim 1, wherein the computer system (500) is further programmed to:
trigger an automatic physical stimulation of the patient (370).

3. The patient monitoring system (400) of claim 1, wherein the computer system (500) is further programmed to:
calculate a length of the apnea event.

4. The patient monitoring system (400) of claim 1, wherein the computer system (500) is further programmed to:
record apnea events for the patient;
send information about historical apnea events to a user interface (600);
calculate aggregate information about the recorded apnea events for the patient; and
send the aggregate information about the recorded apnea events to the user interface (600).

5. The patient monitoring system (400) of claim 4, wherein the computer system is further programmed to receive heart rate and oxygen saturation measurements from the bedside monitoring equipment (420A, 420B, 420C) and to record oxygen saturation and heart rate data associated with the apnea events.

6. The patient monitoring system (400) of claim 1, wherein the computer system (500) is further programmed to receive heart rate and oxygen saturation measurements from the bedside monitoring equipment,
wherein the set of criteria includes heart rate and oxygen saturation criteria.

7. The patient monitoring system (400) of claim 1, wherein the patient is a neonatal infant.

8. A non-transient medium on which is stored software for detecting apnea events in a patient, comprising software that when executed causes a computer system (600) to:
receive chest impedance physiological data and cardiac physiological data from bedside monitoring equipment (420A, 420B, 420C) associated with the patient (310);
model a cardiac phase using the cardiac physiological data (320);
calculate a frequency or time domain approximation of a cardiac artifact based on a representation of the modeled cardiac phase and the chest impedance physiological data (330);
remove the cardiac artifact from the chest impedance physiological data, producing a filtered chest impedance data (340);
calculate a probability of an apnea event based on the filtered chest impedance data (350); and
generate an alarm indication responsive to a set of criteria being met based on the probability of the apnea event over a period of time (360).

9. The non-transient medium of claim 8, wherein the software when executed further causes the computer system to:
trigger an automatic physical stimulation of the patient responsive to the apnea event (370).

10. The non-transient medium of claim 8, wherein the software when executed further causes the computer system to:
calculate a length of the apnea event.

11. The non-transient medium of claim 8, wherein the software when executed further causes the computer system to:
record apnea events for the patient;
send information about historical apnea events to a graphical user interface (600);
calculate aggregate information about the recorded apnea events for the patient; and
send the aggregate information about the recorded apnea events to the graphical user interface (600).

12. The non-transient medium of claim 11, wherein the software when executed further causes the computer system to receive heart rate and oxygen saturation measurements from the bedside monitoring equipment and record oxygen saturation and heart rate data associated with the apnea events.

13. The non-transient medium of claim 8, wherein the software when executed further causes the computer system to:
receive heart rate and oxygen saturation measurements from the bedside monitoring equipment,
wherein the set of criteria includes heart rate and oxygen saturation criteria.

## Patentansprüche

1. Ein Patientenüberwachungssystem (400) zum Erkennen von Apnoe-Ereignissen
bei einem Patienten, umfassend:
ein bettseitiges Überwachungsgerät (420A, 420B, 420C), das dem Patienten zugeordnet ist und dazu ausgelegt ist, physiologische Brustimpedanzdaten und kardiale physiologische Daten zu erzeugen;
ein Computersystem (500), das programmiert ist, um:
die physiologischen Brustimpedanzdaten und die kardialen physiologischen Daten zu empfangen (310);
eine Herzphase unter Verwendung der kardialen physiologischen Daten zu modellieren (320);
eine Frequenz- oder Zeitbereichsapproximation eines Herzartefakts basierend auf einer Darstellung der modellierten Herzphase und der physiologischen Brustimpedanzdaten zu berechnen (330);
das Herzartefakt aus den physiologischen Brustimpedanzdaten zu entfernen, wodurch gefilterte Brustimpedanzdaten erzeugt werden (340);
eine Wahrscheinlichkeit für ein Apnoe-Ereignis basierend auf den gefilterten Brustimpedanzdaten zu berechnen (350); und
eine Alarmanzeige zu erzeugen, wenn eine Reihe von Kriterien erfüllt ist, die auf der Wahrscheinlichkeit des Apnoe-Ereignisses über einen bestimmten Zeitraum basieren (360).

2. Das Patientenüberwachungssystem (400) nach Anspruch 1, wobei das Computersystem (500) ferner programmiert ist, um:
eine automatische physische Stimulation des Patienten (370) auszulösen.

3. Das Patientenüberwachungssystem (400) nach Anspruch 1, wobei das Computersystem (500) ferner programmiert ist, um:
die Dauer des Apnoe-Ereignisses zu berechnen.

4. Das Patientenüberwachungssystem (400) nach Anspruch 1, wobei das Computersystem (500) ferner programmiert ist, um:
Apnoe-Ereignisse des Patienten aufzuzeichnen;
Informationen über bisherige Apnoe-Ereignisse an eine Benutzerschnittstelle (600) zu senden;
aggregierte Informationen über die aufgezeichneten Apnoe-Ereignisse des Patienten zu berechnen; und
die aggregierten Informationen über die aufgezeichneten Apnoe-Ereignisse an die Benutzerschnittstelle (600) zu senden.

5. Das Patientenüberwachungssystem (400) nach Anspruch 4, wobei das Computersystem ferner programmiert ist, um Herzfrequenz- und Sauerstoffsättigungsmesswerte von dem bettseitigen Überwachungsgerät (420A, 420B, 420C) zu empfangen und mit den Apnoe-Ereignissen verbundene Sauerstoffsättigungs- und Herzfrequenzdaten aufzuzeichnen.

6. Das Patientenüberwachungssystem (400) nach Anspruch 1, wobei das Computersystem (500) ferner programmiert ist, um Herzfrequenz- und Sauerstoffsättigungsmesswerte von dem bettseitigen Überwachungsgerät zu empfangen, wobei die Reihe von Kriterien Herzfrequenz- und Sauerstoffsättigungskriterien umfasst.

7. Das Patientenüberwachungssystem (400) nach Anspruch 1, wobei es sich bei dem Patienten um ein Neugeborenes handelt.

8. Ein nichtflüchtiges Medium, auf dem Software zum Erkennen von Apnoe-Ereignissen bei einem Patienten gespeichert ist, umfassend Software, die, wenn sie ausgeführt wird, ein Computersystem (600) dazu veranlasst:
physiologische Brustimpedanzdaten und kardiale physiologische Daten von einem dem Patienten (310) zugeordneten bettseitigen Überwachungsgerät (420A, 420B, 420C) zu empfangen;
eine Herzphase unter Verwendung der kardialen physiologischen Daten zu modellieren (320);
eine Frequenz- oder Zeitbereichsapproximation eines Herzartefakts basierend auf einer Darstellung der modellierten Herzphase und der physiologischen Brustimpedanzdaten zu berechnen (330);
das Herzartefakt aus den physiologischen Brustimpedanzdaten zu entfernen, wodurch gefilterte Brustimpedanzdaten erzeugt werden (340);
eine Wahrscheinlichkeit für ein Apnoe-Ereignis basierend auf den gefilterten Brustimpedanzdaten berechnen (350); und
eine Alarmanzeige erzeugen, wenn eine Reihe von Kriterien erfüllt ist, die auf der Wahrscheinlichkeit des Apnoe-Ereignisses über einen bestimmten Zeitraum basieren (360).

9. Das nichtflüchtige Medium nach Anspruch 8, wobei die Software, wenn sie ausgeführt wird, das Computersystem ferner dazu veranlasst:
eine automatische physische Stimulation des Patienten als Reaktion auf das Apnoe-Ereignis auszulösen (370).

10. Das nichtflüchtige Medium nach Anspruch 8, wobei die Software, wenn sie ausgeführt wird, das Computersystem ferner dazu veranlasst:
die Dauer des Apnoe-Ereignisses zu berechnen.

11. Das nichtflüchtige Medium nach Anspruch 8, wobei die Software, wenn sie ausgeführt wird, das Computersystem ferner dazu veranlasst:
Apnoe-Ereignisse des Patienten aufzuzeichnen;
Informationen über vergangene Apnoe-Ereignisse an eine grafische Benutzerschnittstelle (600) zu senden;
aggregierte Informationen über die aufgezeichneten Apnoe-Ereignisse des Patienten zu berechnen; und
die aggregierten Informationen über die aufgezeichneten Apnoe-Ereignisse an die grafische Benutzerschnittstelle (600) zu senden.

12. Das nichtflüchtige Medium nach Anspruch 11, wobei die Software, wenn sie ausgeführt wird, das Computersystem ferner dazu veranlasst, Herzfrequenz- und Sauerstoffsättigungsmesswerte von dem bettseitigen Überwachungsgerät zu empfangen und mit den Apnoe-Ereignissen verbundene Sauerstoffsättigungs- und Herzfrequenzdaten aufzuzeichnen.

13. Das nichtflüchtige Medium nach Anspruch 8, wobei die Software, wenn sie ausgeführt wird, das Computersystem ferner veranlasst:
Herzfrequenz- und Sauerstoffsättigungsmesswerte von dem bettseitigen Überwachungsgerät zu empfangen,
wobei die Reihe von Kriterien Herzfrequenz- und Sauerstoffsättigungskriterien umfasst.

## Revendications

1. Système de surveillance de patient (400) pour la détection d'événements d'apnée chez un patient, comprenant :
un équipement de surveillance de chevet (420A, 420B, 420C) associé au patient, qui est configuré pour produire des données physiologiques d'impédance thoracique et des données physiologiques cardiaques ;
un système informatique (500) programmé pour :
recevoir les données physiologiques d'impédance thoracique et les données physiologiques cardiaques (310) ;
modéliser une phase cardiaque à l'aide des données physiologiques cardiaques (320) ;
calculer une approximation dans le domaine fréquentiel ou temporel d'un artefact cardiaque sur la base d'une représentation de la phase cardiaque modélisée et des données physiologiques d'impédance thoracique (330) ;
retirer l'artefact cardiaque des données physiologiques d'impédance thoracique, ce qui produit des données d'impédance thoracique filtrées (340) ;
calculer une probabilité d'un événement d'apnée sur la base des données d'impédance thoracique filtrées (350) ; et
générer une indication d'alarme en réponse au fait qu'un ensemble de critères est satisfait sur la base de la probabilité de l'événement d'apnée pendant une période de temps (360).

2. Système de surveillance de patient (400) selon la revendication 1, dans lequel le système informatique (500) est en outre programmé pour :
déclencher une stimulation physique automatique du patient (370).

3. Système de surveillance de patient (400) selon la revendication 1, dans lequel le système informatique (500) est en outre programmé pour :
calculer une durée de l'événement d'apnée.

4. Système de surveillance de patient (400) selon la revendication 1, dans lequel le système informatique (500) est en outre programmé pour :
enregistrer des événements d'apnée pour le patient ;
envoyer des informations concernant des événements d'apnée historiques à une interface utilisateur (600) ;
calculer des informations agrégées concernant les événements d'apnée enregistrés pour le patient ; et
envoyer les informations agrégées concernant les événements d'apnée enregistrés à l'interface utilisateur (600).

5. Système de surveillance de patient (400) selon la revendication 4, dans lequel le système informatique est en outre programmé pour recevoir des mesures de fréquence cardiaque et de saturation en oxygène provenant de l'équipement de surveillance de chevet (420A, 420B, 420C) et pour enregistrer des données de saturation en oxygène et de fréquence cardiaque associées aux événements d'apnée.

6. Système de surveillance de patient (400) selon la revendication 1, dans lequel le système informatique (500) est en outre programmé pour recevoir des mesures de fréquence cardiaque et de saturation en oxygène provenant de l'équipement de surveillance de chevet,
dans lequel l'ensemble de critères inclut des critères de fréquence cardiaque et de saturation en oxygène.

7. Système de surveillance de patient (400) selon la revendication 1, dans lequel le patient est un nouveau-né.

8. Support non transitoire sur lequel est stocké un logiciel pour la détection d'événements d'apnée chez un patient, comprenant un logiciel qui, lorsqu'il est exécuté, amène un système informatique (600) à :
recevoir des données physiologiques d'impédance thoracique et des données physiologiques cardiaques provenant d'un équipement de surveillance de chevet (420A, 420B, 420C) associé au patient (310) ;
modéliser une phase cardiaque à l'aide des données physiologiques cardiaques (320) ;
calculer une approximation dans le domaine fréquentiel ou temporel d'un artefact cardiaque sur la base d'une représentation de la phase cardiaque modélisée et des données physiologiques d'impédance thoracique (330) ;
retirer l'artefact cardiaque des données physiologiques d'impédance thoracique, ce qui produit des données d'impédance thoracique filtrées (340) ;
calculer une probabilité d'un événement d'apnée sur la base des données d'impédance thoracique filtrées (350) ; et
générer une indication d'alarme en réponse au fait qu'un ensemble de critères est satisfait sur la base de la probabilité de l'événement d'apnée pendant une période de temps (360).

9. Support non transitoire selon la revendication 8, dans lequel le logiciel, lorsqu'il est exécuté, amène en outre le système informatique à :
déclencher une stimulation physique automatique du patient en réponse à l'événement d'apnée (370).

10. Support non transitoire selon la revendication 8, dans lequel le logiciel, lorsqu'il est exécuté, amène en outre le système informatique à :
calculer une durée de l'événement d'apnée.

11. Support non transitoire selon la revendication 8, dans lequel le logiciel, lorsqu'il est exécuté, amène en outre le système informatique à :
enregistrer des événements d'apnée pour le patient ;
envoyer des informations concernant des événements d'apnée historiques à une interface utilisateur graphique (600) ;
calculer des informations agrégées concernant les événements d'apnée enregistrés pour le patient ; et
envoyer les informations agrégées concernant les événements d'apnée enregistrés à l'interface utilisateur graphique (600).

12. Support non transitoire selon la revendication 11, dans lequel le logiciel, lorsqu'il est exécuté, amène en outre le système informatique à recevoir des mesures de fréquence cardiaque et de saturation en oxygène provenant de l'équipement de surveillance de chevet, et enregistrer des données de saturation en oxygène et de fréquence cardiaque associées aux événements d'apnée.

13. Support non transitoire selon la revendication 8, dans lequel le logiciel, lorsqu'il est exécuté, amène en outre le système informatique à :
recevoir des mesures de fréquence cardiaque et de saturation en oxygène provenant de l'équipement de surveillance de chevet,
dans lequel l'ensemble de critères inclut des critères de fréquence cardiaque et de saturation en oxygène.
